Europäisches Patentamt

European Patent Office

Office européen des brevets

(11) Publication number: **0 466 251 A1**

# EUROPEAN PATENT APPLICATION

(21) Application number: **91201702.7**

(22) Date of filing: **02.07.91**

(51) Int. Cl.5: **C12N 15/31**, C12P 21/02,
C12N 1/21, C12R 1/465,
C12P 19/02

(30) Priority: **10.07.90 EP 90201851**

(43) Date of publication of application:
**15.01.92 Bulletin 92/03**

(84) Designated Contracting States:
**AT BE CH DE DK ES FR GB GR IT LI LU NL SE**

(71) Applicant: **GIST-BROCADES N.V.**
**Wateringseweg 1 P.O. Box 1**
**NL-2600 MA Delft(NL)**

(72) Inventor: **Luiten, Rudol Gijsbertus Marie**
**Akkerhoornbloem 26**
**NL-2317 KR Leiden(NL)**
Inventor: **Kerkman, Richard**
**Scheepersstraat 60**
**NL-2021 BK Haarlem(NL)**
Inventor: **Bosch, Leendert**
**Hofbrouckerlaan 56**
**NL-2341 LR Oegstgeest(NL)**
Inventor: **Vijgenboom, Erik**
**Rozenoord 62**
**NL-2651 XP Berkel en Rodenrijs(NL)**
Inventor: **Heinstra, Pieter Wilhelmus**
**Hendrikus**
**Eykmanlaan 241**
**NL-3571 JH Utrecht(NL)**
Inventor: **Woudt, Lambertus P.**
**Schouw 2**
**NL-1613 CM Grootebroek(NL)**

(74) Representative: **Huygens, Arthur Victor, Dr. et**
**al**
**c/o Gist-Brocades N.V. Patent & Trademarks**
**Department Wateringseweg 1 PO Box 1**
**NL-2600 MA Delft(NL)**

(54) **Elfamycin-resistant mutants.**

(57) Elfamycin-producing actinomycetes, in particular the mocimycin-producing streptomycetes, are frequently too sensitive for the elfamycin produced by them. This limiting factor for the production of the elfamycin concerned is removed by mutating the gene tuf, encoding the protein EF-Tu, into a gene tufR, encoding a protein EF-TuR, which protein is resistant to the elfamycin concerned. The gene tufR is expressed in host cells which than show an increased resistance to the elfamycins tested.

EP 0 466 251 A1

The present invention relates to elfamycin producing actinomycetes, to a protein EF-Tu (Elongation Factor Tu) of an elfamycin producing actinomycete, to the DNA sequence tuf encoding this protein, to replicable vectors containing this DNA sequence and to actinomycetes transformed with these vectors.

The elfamycins are a group of antibiotics, to which belong Mocimycin (also known as Kirromycin), Dihydromocimycin, N-Methylmocimycin (also known as Aurodox), Kirrothricin, Azdimycin, Efrotomycin, and Pulvomycin. They are produced by bacteria belonging to the order of the Actinomycetales. In particular, the elfamycin antibiotic mocimycin, subject matter of British Patent 1325200, is produced by bacteria belonging to the genus Streptomyces, such as Streptomyces collinus, Streptomyces diastatochromogenes, Streptomyces fradiae and especially Streptomyces ramocissimus.

In practice, the level of production of elfamycins, in particular mocimycin, by the above bacteria is often found to be too low, so as to make their commercial exploitation unattractive.

The antibiotic action of the elfamycins, including mocimycin, is known to be due to their inhibition of EF-Tu (H. Wolf et al., Proc. Natl. Acad. Sci. USA, 75 (1978) 5324-5328).

The Polypeptide Chain Elongation Factors (EF) are essential for cellular protein synthesis. The type designated EF-Tu occurs in all prokaryotic cells, including gram-negative bacteria such as Escherichia coli and gram-positive bacteria such as those belonging to the order of Actinomycetales. Different organisms have similar, but not identical, EF-Tu. The DNA sequence encoding EF-Tu has been designated the tuf gene.

It was further found by C. Glöckner and H. Wolf (FEMS Microbiology Letters 25 (1984) 121-124), that the EF-Tu isolated from all tested mocimycin producing strains of the genus Streptomyces was sensitive to relatively low concentrations of elfamycin in a cell-free protein synthesizing system. On the other hand, these authors found the EF-Tu isolated from the kirrothricin producing Streptomyces cinnamomeus and from the efrotomycin-producing Streptomyces lactamdurans (recently renamed Nocardia lactamdurans) to be relatively resistant not only to the endogenous antibiotic but also to mocimycin. These authors suggested that the sensitivity of the EF-Tu of the elfamycin producing strains to their own elfamycin is the limiting factor of their production capacity. They speculated that strains such as Streptomyces cinnamomeus and Streptomyces lactamdurans may be suitable sources of mutants with increased productivity because they tolerate high antibiotic levels in the cell.

Mutagenesis, by chemical mutagenic compounds, of originally elfamycin sensitive EF-Tu into EF-Tu exhibiting an increased resistance to elfamycin, was described by E. Fischer et al. (Proc. Natl. Acad. Sci. USA, 74 (1977) 4341-4345) in a laboratory strain of E. coli having altered membrane permeability, and also by J.A.M. van de Klundert et al. (FEBS Letters 81 (1977) 303-307). The first-mentioned authors report the mutant E. coli to be deficient in growth capacity (in the absence of elfamycin), when compared to the parent E. coli. The mutation leading to the increased elfamycin resistance of E. coli EF-Tu was found to be a change of an alanine residue at position 375 to either valine or threonine (F.J. Duisterwinkel et al., EMBO J. 3 (1984) 113-120). No other elfamycin resistant EF-Tu proteins have been reported from other gram-negative bacteria; an elfamycin resistant EF-Tu from the gram-positive bacterium Bacillus subtilis has been identified, but not characterized at the molecular level (I. Smith and P. Paress, J. Bacteriol. 135 (1978) 1107-1117). No such mutations have been described in any actinomycete, especially in streptomycetes, in particular in mocimycin producing streptomycetes, more in particular in Streptomyces ramocissimus.

In the two above-mentioned publications about chemical mutagenesis of E. coli strains leading to an increased resistance to elfamycin, it is disclosed that E. coli has two closely related but distinct EF-Tu proteins, originating from two distinct tuf genes. Since elfamycin inhibits the activity of sensitive EF-Tu by binding it irreversibly to the ribosomes, it follows on theoretical grounds that an elfamycin resistant mutant of E. coli has to have either the two EF-Tu proteins both mutated and active, or only one of them mutated and active, the other one then being non-active. This was confirmed by in vitro experiments.

In streptomycetes, in particular in Streptomyces ramocissimus the present inventors have identified three closely related tuf-genes. On further investigation it was discovered that one of those is mainly expressed in the vegetative mycelium of the streptomycete. The protein products of both other genes constitute approximately 5 percent of the amount of the main EF-Tu species. This has specifically been observed in Streptomyces ramocissimus. A similar protein pattern was found in Streptomyces collinus, and Streptomyces goldiniensis.

In contrast to Escherichia coli, streptomycetes have the capacity to undergo a complex morphological and biochemical differentiation towards spore formation. It is therefore conceivable that during the sporulation and subsequent germination process (one of) the minor vegetative EF-Tu species becomes the main active EF-Tu. This differential expression would then be analogous to e.g. the expression of different sigma factors observed in B. subtilis (R. Losick et al., Ann. Rev. Genetics 20 (1986) 625-669) and S. coelicolor A3-(2) (M.J. Buttner, Molecular Microbiol. 3 (1989) 1653-1659) directing the transcription of developmentally regulated sets of genes. Differential expression of EF-Tu encoding genes may be required to adapt the translation machinery to specific requirements of the developmental phase.

Even though the level of expression of the two minor EF-Tu species in the vegetative mycelium is low, it is still possible that, in analogy to the situation in Escherichia coli, they convey the dominance of the elfamycin sensitivity even if the major EF-Tu protein is rendered elfamycin resistant; the relative level of elfamycin sensitive EF-Tu versus elfamycin resistant EF-Tu at which an elfamycin resistance phenotype becomes apparent is unknown. For the purpose of the present invention however, streptomycetes, in particular Streptomyces ramocissimus is considered to have one major EF-Tu.

It has now been found possible to modify the elfamycin sensitive EF-Tu protein of an elfamycin producing actinomycete, in particular a mocimycin producing streptomycete, more in particular one belonging to the species Streptomyces ramocissimus, thereby conferring to this protein an increased resistance to the elfamycin produced by this bacterium. This has been found possible to achieve by mutagenesis. In particular, the present inventors have achieved the mutagenesis by using site-directed mutagenesis techniques for modifying the original gene tuf, encoding the elfamycin sensitive EF-Tu, to a novel gene tufR, encoding a novel protein EF-TuR having an increased resistance to the elfamycin.

The present invention therefore provides proteins EF-TuR, characterized in that they have been derived from an elfamycin producing actinomycete and made resistant to the elfamycin by mutagenesis, in particular site-directed mutagenesis.

The invention further provides various DNA sequences tufR, encoding said proteins EF-TuR.

The invention still further provides vectors, containing said DNA sequences. Such vectors are replicable and/or capable of integrating into the chromosomal DNA sequence of an elfamycin producing actinomycete.

The invention yet further provides an elfamycin producing actinomycete, comprising a DNA sequence tufR instead of the DNA sequence tuf. Such actinomycetes have been found to possess a substantially increased resistance to the elfamycin. The increased resistance of the EF-Tu protein to elfamycin removes a limiting factor in the elfamycin production. The expression of the elfamycin resistant tuf genes is found to influence the growth of the transformed strains.

The invention further provides processes for the preparation of said DNA sequences, vectors, and actinomycete.

## Description of the drawings

In the drawings the following abbreviations and symbols are used:

**bla** : ampicillin resistance gene (**bla~** if inactive);
**bla\*** : position of translation stopcodon in **bla~**;
**cat** : chloramphenicol resistance gene (**cat~** if inactive);
**cat\*** : position of translation stopcodon in **cat~**;
**tsr** : thiostrepton resistance gene;
**ori322** : replication origin derived from plasmid pBR322;
**orif1** : replication origin derived from phage f1;
**rep660** : replication origin region derived from plasmid pMT660;
**Srtuf** : S. ramocissimus tuf gene, **Srtuf 3'** if only the 3' coding region of the gene is present;
**Plac** : E. coli lac operon promoter.

### Figure 1

The DNA sequence of the S. ramocissimus tuf1 gene and the amino acid sequence of the S. ramocissimus EF-Tu1 protein derived therefrom (also shown as SEQ ID NO : 1).

EF-TuR is characterized here by replacement of the amino acid alanine at position 378 by valine or threonine, respectively.

The gene tufR is characterized here in that the codon encoding alanine at position 378 (GCC) is changed to codons encoding valine, threonine, proline, or phenylalanine.

### Figure 2

a. Map of plasmid pUSrT1.
b. Map of expression plasmid pUSrT1-1. In plasmids pUSrT1V-1, pUSrT1T-1, pUSrT1P-1, and pUSrT1F-1, Ala378 is replaced by valine, threonine, proline, or phenylalanine, respectively.

Figure 3

a. Map of plasmid pMaSrT1. In plasmids pMaSrT1V, pMaSrT1T, pMaSrT1P, and pMaSrT1F, Ala378 is replaced by valine, threonine, proline, and phenylalanine, respectively.
b. Map of plasmid pMcSrT1.

Figure 4

a. Graphic representation of residual activities of SrEF-Tu and SrEF-Tu mutants A378V and A378T in an in vitro polyphenylalanine synthesizing system, in the presence of different concentrations of mocimycin.
b. Graphic representation of the time course of incorporation of $^3$H-phenylalanine in an in vitro polyphenylalanine synthesizing system by SrEF-Tu and SrEF-Tu mutants A378V and A378T in the presence of 16 mg/l mocimycin.

Figure 5

a. Analysis of the mutant SrEF-Tu1 proteins A378V and A378T in the elfamycin binding assay. Lanes 1 and 2: wild-type SrEF-Tu, lanes 3 and 4: SrEF-Tu A378T, lanes 5 and 6: SrEF-Tu A378V. In lanes 2, 4, and 6 the indicated SrEF-Tu protein have been pre-incubated with 25 $\mu$M aurodox.
b. Analysis of the mutant SrEF-Tul proteins A378P and A378F in the elfamycin binding assay. Lanes 1 and 2: wild-type SrEF-Tu, lanes 3 and 4: SrEF-Tu A378F, lanes 5 and 6: SrEF-Tu A378P. In lanes 2, 4, and 6 the indicated SrEF-Tu protein have been pre-incubated with 25 $\mu$M Aurodox.

Figure 6

a. Map of plasmid pUt18.
b. Map of plasmid pStT1-1. In plasmids pStT1V-1 and pStT1T-1, Ala378 is replaced by valine and threonine, respectively.

Figure 7

a. Map of plasmid pStT1ΔS. In plasmids pStT1VΔS and pStT1TΔS, Ala378 is replaced by valine and threonine, respectively.
b. Map of plasmid pMTST1ΔS. In plasmids pMTST1VΔS and pMTST1TΔ S, Ala378 is replaced by valine and threonine, respectively.

Figure 8

a. Map of the S.ramocissimus CBS 190.69 chromosomal tuf locus.
b. Map of the S.ramocissimus tuf locus in which plasmid pMTST1VΔS is integrated via homologous recombination.
c. Map of the S.ramocissimus R1V chromosomal tufR locus.

Figure 9

Analysis of the SrEF-Tu from S. ramocissimus strain R1V with respect to elfamycin binding. Samples were loaded on the native gel as follows: Lanes 1 and 2: wild-type SrEF-Tu isolated from E. coli JM101-[pUSrT1-1], lanes 3 and 4: SrEF-Tu isolated from S. ramocissimus CBS 190.69, lanes 5 and 6: SrEF-Tu isolated from S. ramocissimus strain R1V. In lanes 1, 4, and 6, the indicated SrEF-Tu proteins were pre-incubated with 25 $\mu$M Aurodox.

Detailed description of the invention

Elfamycin producing species can be found among the Actinomycetes. Preferably Streptomycetes are used. Examples are the mocimycin producing streptomycetes Streptomyces collinus, Streptomyces diastatochromogenes, Streptomyces fradiae, and Streptomyces ramocissimus. Most preferably S.ramocissimus is used.

Elongation factor Tu (EF-Tu) can be isolated in a number of ways. For example different combinations of general protein purification techniques known in the art, such as stepwise ammonium sulphate precipitation, gel filtration, and ion-exchange chromatography can be used. Application of this approach for the purification of EF-Tu protein have been described by D. Miller and H. Weissbach (Arch. Biochem. Biophys. 141 (1970) 26-37), K.-I. Arai et al. (J. Biol. Chem. 247 (1972) 7029-7037), and R. Leberman et al. (Anal. Biochem. 104 (1980) 29-36). A preferred isolation procedure for EF-Tu is the following. After culturing S. ramocissimus the mycelium is harvested by centrifugation. The mycelium is resuspended and sonicated. After differential centrifugation to remove the ribosomes, the protein is further purified by affinity chromatography (G. Jacobson and J. Rosenbusch, FEBS Lett. 79 (1977) 8-10); for this purpose GDP-AH-Sepharose is especially useful. After purification the protein is further characterized by GDP exchange analysis (H. Weissbach et al., Arch. Biochem. Biophys. 137 (1970) 262-269) and by its ability to promote EF-Tu dependent peptide synthesis in a cell-free extract, e.g. as described by C. Glöckner and H. Wolf (cited above). Further characterization can be performed by determination of the amino acid composition and (partial) amino acid sequence of the protein.

Susceptibility of the isolated EF-Tu to elfamycin is tested in elfamycin binding studies (G. Chinali et al., Eur. J. Biochem. 75 (1977) 55-65,) and in studies on the inhibition of EF-Tu dependent peptide synthesis (C. Glöckner and H. Wolf, cited above). Still another direct elfamycin binding assay has been developed in which the capacity of the EF-Tu protein to bind elfamycins can be visualized by a change in EF-Tu protein migration in the presence of the elfamycin by non-denaturing PAGE (polyacrylamide gel electrophoresis). Upon elfamycin binding to EF-Tu.GDP, a ternary complex is formed which is more negatively charged than the binary EF-Tu.GDP complex (B. Kraal et al, 1989, in The Guanine-Nucleotide Binding Proteins, pp 121-129, Plenum Press, New York). Consequently elfamycin binding, using e.g. aurodox, to Escherichia coli wild-type EF-Tu increases the migration distance into a non-denaturing polyacrylamide gel. Both the methods of peptide synthesis inhibition and visualization of elfamycin binding are the preferred techniques to determine the effect of elfamycin binding on EF-Tu functioning. These susceptibility assays are important if increased elfamycin resistance of EF-Tu has to be established.

Several ways are possible to obtain mutants of EF-Tu exhibiting an increased resistance to elfamycin. One of them is mutagenesis of the parent microorganism. This can be performed by for example chemicals, such as ethyl methane sulphonate and N-methyl-N'-nitro-N-nitrosoguanidine, or by UV irradiation. Subsequent selection for increased elfamycin resistance can yield strains that contain EF-Tu with an increased resistance to elfamycin. This can be tested by isolating the protein and performing on it elfamycin binding studies, and by EF-Tu dependent peptide synthesis as described above. Another way of performing the mutagenesis is on the cloned gene coding for the EF-Tu. In this approach it is possible to randomly mutagenize this gene by chemical (R. Myers et al., Science 229 (1985) 242-247) or enzymatic means (P. Lehtovaara et al., Protein Engineering 2 (1988) 63-68), or to focus mutagenesis on one or more specific regions/nucleotides of the gene (site-directed mutagenesis). Site-directed mutagenesis is the preferred embodiment of the present invention.

For cloning the gene encoding the EF-Tu, chromosomal DNA from the relevant elfamycin producing species is isolated and inserted in a suitable vector. Possible vectors are among others plasmids, phages, and cosmids. If necessary, expression vectors can be used. The clones containing the tuf genes can be selected via hybridization with synthetic probes, which are synthesized according to previously determined protein or partial protein sequences. It is also possible to use tuf genes isolated from other species as hybridization probes, provided that there is sufficient similarity between the two genes. It is assumed that when 80% identity exists at the protein level there will be enough identity at the DNA level to detect homologous genes by hybridization. Hence genes from other species that can be found by hybridizaton and that encode a protein having elongation factor activity are also covered by the present invention. Upon cloning in an expression vector it also becomes possible to screen the DNA library thus obtained using antibodies specific to EF-Tu. Preferably the chromosomal DNA from S. ramocissimus is isolated (as described by D. Hopwood et al. (1985) Genetic Manipulation of Streptomyces: A Laboratory Manual, John Innes Foundation, Norwich) and cloned in a plasmid such as pUC8 or pUC18. Selection of one tuf gene is performed using the Hpal/NruI fragment of the E. coli tufA gene as a hybridization probe (T. Yokota et al., Gene 33 (1980) 25-31). At a later stage the first S. ramocissimus tuf gene was used as a probe. In this way three tuf genes were detected, cloned, and then sequenced using the Sanger method (Proc. Natl. Acad. Sci. USA 74 (1977) 5463-5467). By using specific probes derived from these three sequences in a Northern blotting experiment, transcription of only one of the tuf genes was detected during vegetative growth of S. ramocissimus. This main functional S. ramocissimus tuf gene (Srtuf1) was found to be located on a 2.8 kb BglIII chromosomal restriction fragment. Furthermore, using specific antibodies, it was found that the protein encoded by the Srtuf1 gene was approximately 20 times more abundant than the proteins encoded by the other two genes. The latter genes, called Srtuf2 and Srtuf3, were encoded on a 3.0 kb BamHI fragment and a 4.2 kb PstI fragment, respectively.

In order to test whether the elfamycin resistance of the SrEF-Tu1 could be improved, site-directed mutagenesis was applied on the Srtuf gene. From comparison of EF-Tu sequences found in different species it is possible to make a prediction which amino acids are important. Other ways to achieve this may be the analysis of the three-dimensional structure of the protein, inhibitor studies or enzymatic mechanism studies. From the information thus obtained specific mutations can be proposed.

For the elfamycin resistant E. coli strains mentioned above it has been found that replacement of the amino acid alanine at position 375 of the E. coli EF-Tu protein by valine or threonine results in an EF-Tu molecule with an increased resistance to elfamycin (F. Duisterwinkel et al., FEBS Letters 13 (1981) 89-93, F. Duisterwinkel et al., EMBO J. 3 (1984) 113-120).

Several techniques can be employed to introduce similar mutations into the DNA encoding the EF-Tu protein of S. ramocissimus. In a preferred embodiment the pMa-c vector system and E. coli host strains WK6 and WK6mutS are employed (P. Stanssens et al. Nucl. Acid. Res. 17, (1989) 4441-4454), in combination with gapped-duplex mutagenesis (W. Kramer et al. Nucl. Acid. Res. 12 (1984) 9441-9456). Specifically synthetic oligonucleotide probes were designed and used to mutagenize the alanine at position 378 in EF-Tu from S. ramocissimus to valine (A378V), threonine (A378T), proline (A378P), or phenylalanine (A378F). Other possibilities for mutation are yet other amino acid residues at position 378, or e.g. mutation of glutamic acid at position 360 into phenylalanine.

To obtain the modified protein the mutated gene can be expressed in any suitable host; examples are given of expression in E. coli and in S. ramocissimus.

The sensitivity of SrEF-Tu mutants A378V and A378T to elfamycin was tested by in vitro studies. Both parent and mutant S. ramocissimus EF-Tu, after transformation of the respective cloned genes, were expressed in an E. coli strain encoding an elfamycin resistant EF-Tu. Cell-free extracts of these transformants were subsequently tested for elfamycin sensitivity of the translation apparatus, using a variation on the procedure described by C. Glöckner and H. Wolf (cited above). It was found that the SrEF-Tu mutants A378V and A378T had a residual activity of 50% at an elfamycin concentration of 160 mg/l. The parent SrEF-Tu reached 50% residual activity already at 1.6 mg/l. Therefore, elfamycin resistant EF-Tu proteins are considered to be proteins with a residual activity of 50% at an elfamycin concentration of at least 2 mg/ml, when tested in the above assay.

All mutant EF-Tu proteins obtained were tested through direct binding studies visualized by a change in migration in non-denaturing PAGE upon elfamycin binding. Each mutant EF-Tu (A378V, A378T, A378P, and A378F) proved to be unable to bind the elfamycin in this assay.

The mutated genes are introduced into the mocimycin producing host. In a preferred embodiment this is S. ramocissimus. Preferably, the mutated gene is integrated into the chromosome. To that purpose an integration vector can be used, having homology with the tuf gene locus. Integration is then preferably performed at this locus, whereby the parent gene is replaced by the mutated gene. The mutated gene can also be inserted into the chromosome at other loci of choice, preferably loci where the expression level of the encoded protein is high. For high level expression of the protein, plasmid location is also possible and can be advantageous. In the latter two cases (insertion of the tufR at another locus than the tuf gene and plasmid encoded tufR) it is essential that the parent gene is inactivated by mutation, e.g. deletion of the complete gene or a part thereof or of the regulating sequences.

The S. ramocissimus strain in which the chromosomal Srtuf1 gene was replaced by tufR was found to have its resistance level towards elfamycin increased more than 5-fold in the vegetative mycelial growth phase. In addition resistance towards the effects of exogenous elfamycins on sporulation and germination of spores was equally increased. An elfamycin resistant Streptomycete is defined as a strain characterized in that its spores germinate and grow in YMG medium containing yeast extract 4 g/l, malt extract 10 g/l and glucose 4 g/l, in the presence of at least 0.2 g elfamycin /l preferably 0.2-1.0 g elfamycin /l.

In contrast to Escherichia coli no adverse effects of the EF-Tu mutation on the growth rate of the elfamycin resistant Streptomyces ramocissimus was observed.

As demonstrated the EF-TuR proteins of this invention will give rise to strains with an increased resistance against elfamycins. Elfamycin production will be increased or at least if measures are taken to increase the elfamycin production the strains containing the modified proteins will be capable of increased elfamycin production.

The following examples will illustrate the invention, without in any way limiting its scope.

In the examples, unless otherwise specified, all procedures for making and manipulating recombinant DNA using E. coli as a host were carried out by standardized procedures described by T. Maniatis et al. (1982, Molecular Cloning: A Laboratory Manual. Cold Spring Harbor Laboratory, Cold Spring Harbor, N.Y.)

Example 1

Isolation and characterization of Elongation Factor Tu from S. ramocissimus (SrEF-Tu)

S. ramocissimus CBS 190.69 was cultured in liquid S-medium (M. Okanishi et al., J. Gen. Microbiol. 80 (1974) 389-400) for 72 hrs at 30 °C. Mycelium was harvested by centrifugation and resuspended in icecold standard buffer (10 mM Tris/HCl pH 7.8, 60 mM NH₄Cl, 10 mM Mg-acetate, 1 mM DTT, 0.1% PMSF). The suspension was sonicated at 0 °C with 10 bursts of 45 seconds, allowing 15 seconds in between for cooling. The sonicated suspension was centrifuged at 30000 g for 15 minutes. The ribosomes still present in the resulting S-30 extract were pelleted by centrifugation for 3 hr at 100000 g. The supernatant of this centrifugation was regarded as the S-100 fraction of S. ramocissimus mycelium.

SrEF-Tu was purified by affinity chromatography on GDP-AH-Sepharose (G. Jacobson and J. Rosenbusch, cited above). This procedure yielded a single component protein preparation as judged by SDS-PAGE. The protein migrated with an apparent molecular weight of 50 kD, whereas E. coli EF-Tu migrates at 45 kD. The purified protein was identified as S. ramocissimus EF-Tu (SrEF-Tu) by analysis of the protein by GDP exchange experiments (H. Weissbach et al., cited above) and by its ability to promote EF-Tu dependent poly(U) directed synthesis of polyphenylalanine using E. coli ribosomes.

Both elfamycin binding studies (G. Chinali et al., cited above) and the inhibition by added elfamycin of the in vitro poly(U) translation system directed by SrEF-Tu indicated that SrEF-Tu is elfamycin sensitive (see Example 5, and also C. Glöckner and H. Wolf, cited above).

The purified SrEF-Tu was used to raise polyclonal antibodies in rabbits according to standard techniques.

Example 2

Identification, isolation and characterization of the S. ramocissimus tuf genes

The procedure used to isolate S. ramocissimus CBS 190.69 chromosomal DNA was essentially that described by D. Hopwood et al. (cited above). Southern blotting experiments of this DNA, digested with restriction enzymes, showed that an E. coli tufA probe (HpaI/NruI fragment, T. Yokota et al., cited above) hybridized strongly with a BglII fragment of approximately 3.0 kb and less strongly but very specifically to a 3.0 kb BamHI fragment and a 4.2 kb PstI fragment.

For cloning of the strongly hybridizing DNA fragment, S. ramocissimus chromosomal DNA was digested to completion with BglII and ligated with BamHI digested plasmids pUC8 (J. Vieira and J. Messing, Gene 19 (1982) 259-268) and pUC18 (C. Yanisch-Perron et al. Gene 33 (1985) 103-119), respectively. The host for transformation was E. coli strain JM101 (J. Messing, Recombinant DNA Technical Bulletin 2 (1979) 43-48).

A sib-selection procedure was applied to screen pools of transformants for the presence of S. ramocissimus tuf sequences. With this procedure the initial selection by Southern hybridization is applied to plasmids isolated from a pool of transformants. A positive pool is successively reduced in size and in each step the total plasmid population of the pool is screened by Southern hybridization. Finally plasmids isolated from single transformants are analyzed.

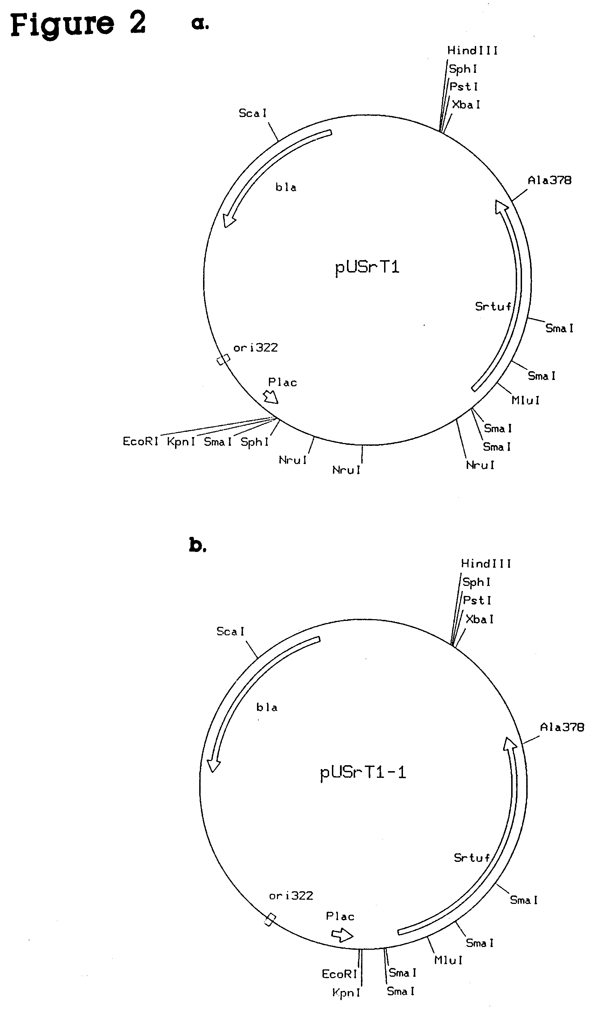

In the sib-selection procedure to isolate the S. ramocissimus tuf gene, plasmid DNA from 11 pools of 50-70 transformants each was isolated and electrophoresed on agarose gels. Southern hybridization of these DNA preparations with the E. coli tufA probe revealed one positive pool. Successive reduction of the pool size resulted in one positive recombinant pUC18 plasmid containing a BglII insert of 2.8 kb. This plasmid was designated pUSrT1 (Figure 2a).

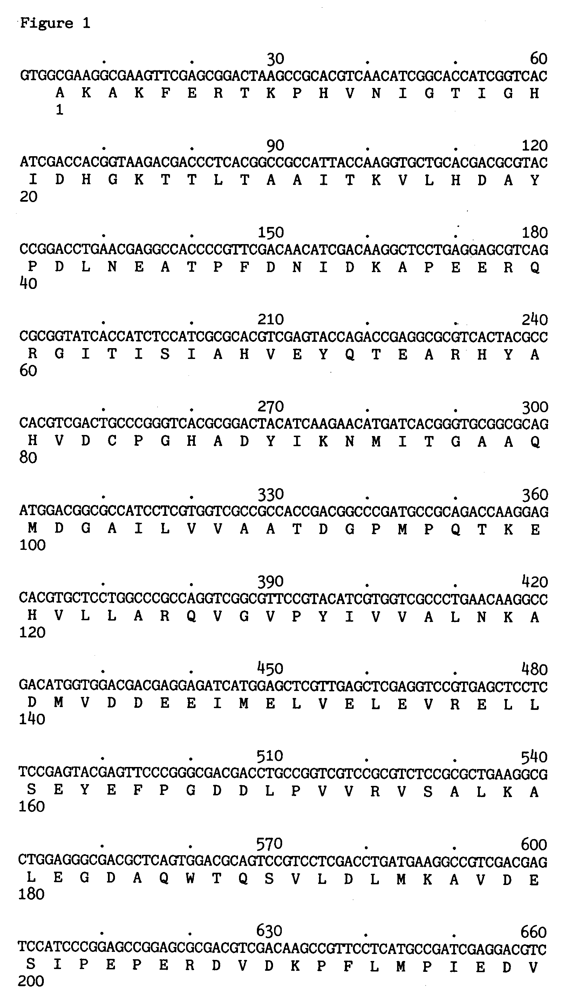

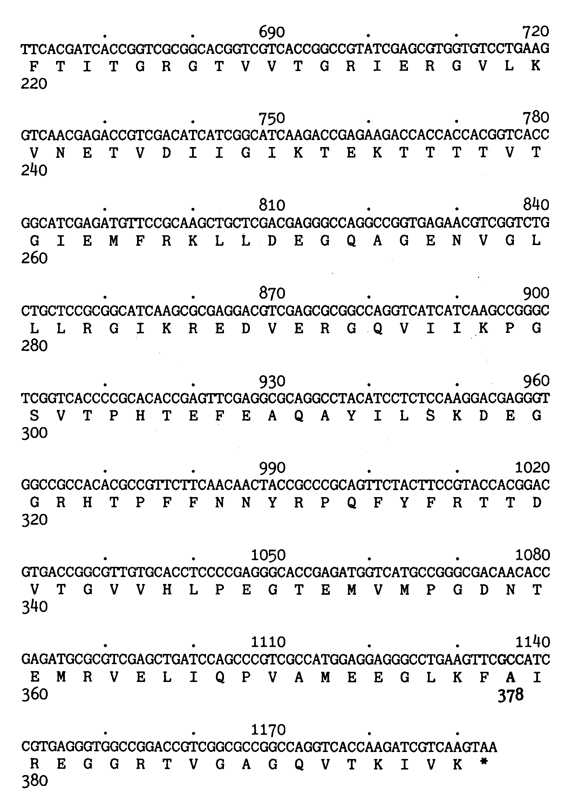

The complete 2.8 kb fragment was sequenced on both strands using the chain termination method of Sanger et al. (cited above), and M13mp18 or M13mp19 phages (C. Yanisch-Perron, cited above) as vector. Analysis of the sequence revealed an open reading frame of 1191 bp, encoding a protein of 397 amino acids, including the N-terminal methionine (Figure 1; SEQ ID 1). The protein sequence derived from this open reading frame showed a 74% homology with E. coli EF-Tu. The gene cloned on pUSrT1 was considered to be the S. ramocissimus EF-Tu encoding gene Srtuf1.

Similarly, using the Srtuf1 gene as a hybridization probe, the 3.0 kb BamHI fragment harboring the Srtuf2 gene and the 4.2 kb PstI fragment harboring the Srtuf3 gene were cloned and the nucleotide sequences of the coding regions determined. The coding sequences of Srtuf2 and Srtuf3 and the derived amino acid sequences of the encoded products SrEF-Tu2 and SrEF-Tu3 are listed as SEQ ID 2 and SEQ ID 3, respectively. SrEF-Tu2 has 71% and SrEF-Tu3 64% of its amino acid residues identical to E. coli EF-Tu.

Example 3

Heterologous expression of the Srtuf1 gene in E. coli

For an independent identification and characterization of the S. ramocissimus EF-Tu protein, the cloned Srtuf1 gene was expressed in E. coli JM101. Expression was obtained by placing the Srtuf gene downstream of the inducible lac promoter on the E. coli plasmids pUC18 as follows:

The NruI/XbaI fragment of pUSrT1 containing the Srtuf gene was isolated, ligated with SmaI/XbaI digested pUC18, and transformed to E. coli JM101 yielding plasmid pUSrT1-1 (Figure 2b).

Growth of E. coli JM101 transformed with pUSrT1-1 and induction of the lac promoter was achieved by culturing the transformants for 16 hrs at 37°C in LB-medium supplemented with 100 $\mu$g/ml ampicillin and 0.5 mM IPTG.

Total protein of these cells was analyzed using SDS-PAGE. This revealed the presence of a new protein species in the transformed E. coli. This protein comigrated with purified SrEF-Tu, and reacted strongly with SrEF-Tu antibodies (see Example 1) in Western blotting experiments.

This experiment thus identified the gene present on pUSrT1-1, as the Srtuf1 gene encoding a protein called SrEF-TuI.

For purification of SrEF-Tu1 an S-100 fraction of E. coli JM101/pUSrT1-1 cells was prepared (Example 1), stabilized by the addition of GDP to 25 $\mu$M and passed through a GDP-AH Sepharose column. Under these conditions the E. coli EF-Tu is bound to the column, whereas the SrEF-Tu1 protein passes through. The GDP-stabilized eluate was then applied to a Dyematrix REd-A column (Amicon). After elimination of unbound protein, the SrEF-Tu1 was eluted at approximately 0.45 M NaCl by applying a linear salt gradient from 0 to 1.5 M NaCl.

Example 4

Site directed mutagenesis of Srtuf1

For site directed mutagenesis of the Srtuf1 gene the pMa-c vector system and E. coli host strains WK6 and WK6mutS (P. Stanssens et al., cited above) were employed in combination with the gapped-duplex mutagenesis protocol (W. Kramer et al., cited above).

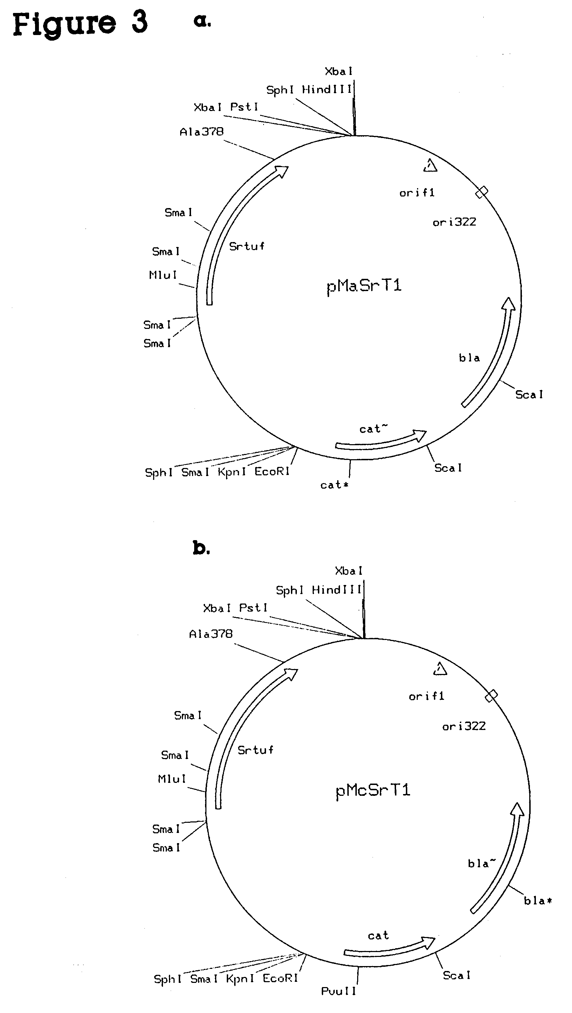

pUSrT1 was digested with EcoRI and HindIII and the Srtuf gene containing fragment was ligated into EcoRI and HindIII digested pMa6 and pMc6 yielding plasmids pMaSrT1 and pMcSrT1, respectively (Figure 3). pMa6 and pMc6 are derivatives of plasmids pMa5-8 and pMc5-8 (P. Stanssens et al., cited above), lacking the PstI site within the $\beta$-lactamase gene.

The mutagenesis and mutant selection procedure was performed using plasmids pMasrT1 and pMcSrT1, essentially as described by P. Stanssens et al. (cited above). In short, single-stranded DNA was prepared from plasmid pMcSrT1 by infection of pMcSrT1 containing E. coli JM101 cells with phage M13KO7. For formation of the gapped duplex, single-stranded pMcSrT1 was combined with the larger MluI/XbaI fragment of pMaSrT1 and either synthetic oligonucleotide 1 (SEQ ID 4), or synthetic oligonucleotide 2 (SEQ ID 5) to mutate position 378 (alanine) of the SrEF-Tu1 protein to valine and threonine. The mutant proteins were designated SrEF-Tu A378V and A378T, respectively. After gap-filling and ligation using DNA polymerase I (large fragment) and T4-DNA ligase, the samples were transformed to E. coli WK6mutS, while selecting for ampicillin resistance. Next, plasmid DNA was isolated from pooled WK6mutS transformants and introduced into E. coli strain WK6. Individual ampicillin resistant WK6 transformants were subsequently infected with M13KO7 as described above in order to obtain plasmid DNA in single-stranded form. Nucleotide sequence analysis (see Example 2) was used to identify clones containing the desired mutation, and to ascertain that no secondary mutations had been introduced within the gap during the mutagenesis procedure.

Plasmids containing the respective desired mutations were recovered and designated pMaSrT1V and pMaSrT1T (Figure 3).

Similarly, the mutations A378P (proline) and A378F (phenylalanine) were introduced using mutagenic oligonucleotides 3 (SEQ ID 6) and 4 (SEQ ID 7), respectively. Plasmids obtained by these experiment were designated pMaSrT1P and pMaSrT1F.

Example 5

Properties of SrEF-Tu mutants A378V and A378T in an in vitro peptide synthesis assay

In order to obtain expression of the mutant Srtuf genes, the larger MluI/XbaI fragment of pUSrT1-1 was ligated with the smaller MluI/XbaI fragment of both pMaSrT1V and pMaSrT1T, yielding plasmids pUSrT1V-1 and pUSrT1-1, respectively (Figure 2).

Plasmids pUSrT1-1, pUSrT1V-1 and pUSrT1T-1 were transformed to E. coli PM1455 (tufA, tufB::Mu, rpoB, recA56; P. van der Meide et al., Eur. J. Biochem. 130 (1983) 409-417); this strain has only one active tuf gene, which encodes an elfamycin resistant EF-Tu. The respective E. coli PM1455 transformants were grown as described in Example 3, and an S-30 extract was prepared essentially as described in Example 1. One ml of the extract was applied on a 10 ml Sephadex G-25 column (2 g of Sephadex G-25) of 15-20 cm length (10 ml pipet). The column was eluted with standard buffer (Example 1) and fractions of 5 drops (500-700 $\mu$l) were collected. The first 4 fractions having absorbance at 260 nm were pooled. This crude pooled fraction was used for promoting in vitro poly(U) directed poly(phe) synthesis as follows.

At 0°C an incubation mixture was prepared consisting of 40 mM Tris-acetate pH 7.6, 10 mM Mg-acetate, 60 mM NH$_4$Cl, 5 mM $\beta$-mercaptoethanol, 1 mM ATP, 0.025 mM GTP, 2.5 mM phosphoenol-pyruvate, 0.25 $\mu$g/ml pyruvate kinase, 0.8 mg/ml tRNA, 0.1 mg/ml poly(U), 95 $\mu$M phenylalanine, and 3 $\mu$Ci/ml $^3$H-phenylalanine (57 Ci/mmol). To 0.6 ml of this incubation mixture 0.12 ml crude extract was added. Subsequently 50 $\mu$l samples were incubated at 37°C. Incubation mixtures were processed as follows: 150 $\mu$l 100 mM NaOH was added and incubation was prolonged for 5 minutes at 37°C. Next 800 $\mu$l 5% trichloroacetic acid (TCA) was added and the samples were stored at 0°C for 5 minutes. The precipitate was filtered over GFC filters (Whatman), washed three times with 5% TCA and once with 96% ethanol. Then the filters were dried for 30 minutes at 80°C, 2 ml xylene scintillation fluid was added to each filter. Incorporation of $^3$H-phenylalanine was analyzed in a liquid scintillation counter.

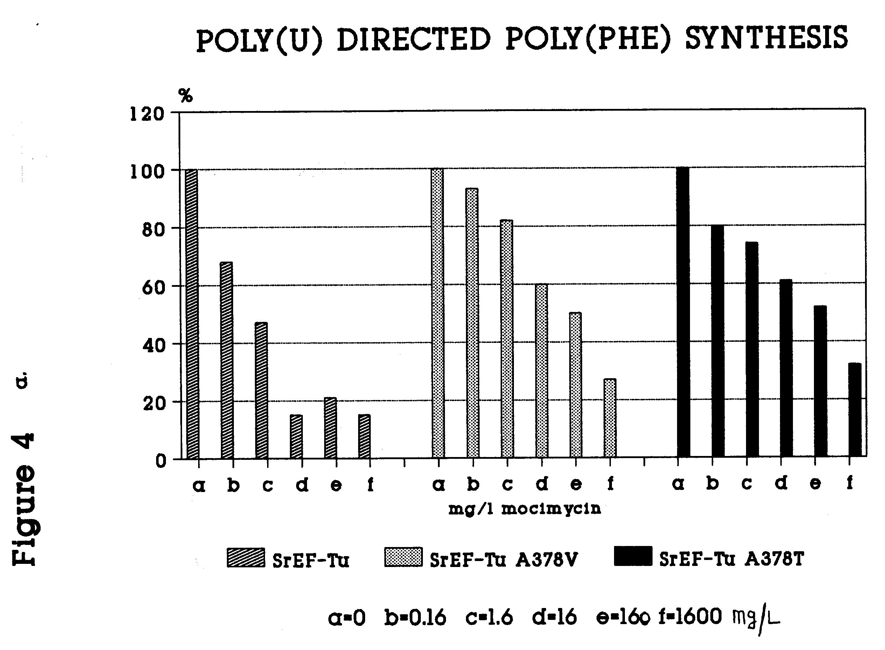

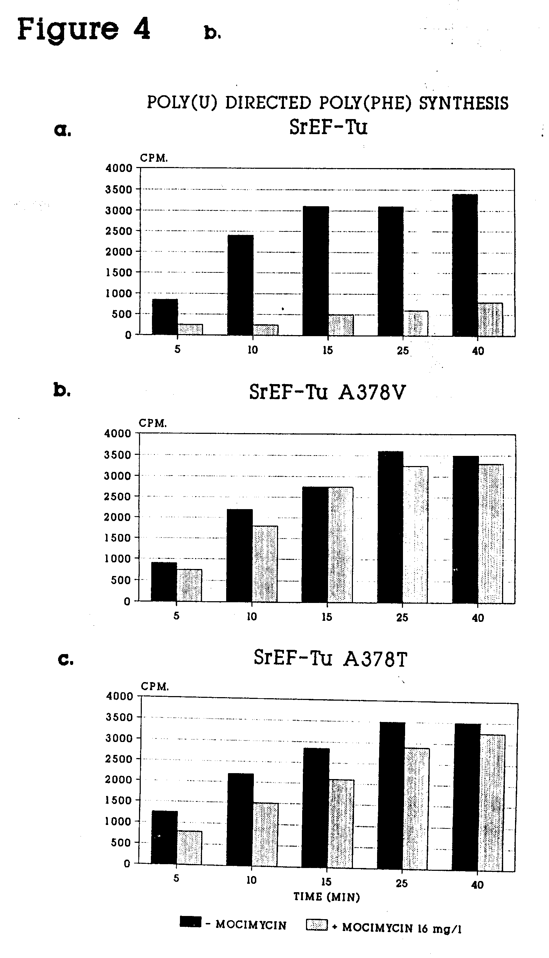

In different experiments either the elfamycin concentrations or the incubation times were varied as indicated in Figure 4. The result of the first experiment is displayed in Figure 4a. Increasing amounts of mocimycin were added to incubation mixtures in parallel poly(phe) synthesis experiments using the S-30 extract mentioned above. Reaction times were kept constant at 10 minutes. Both SrEF-Tu mutants A378V and A378T displayed a residual activity of 50% in the in vitro poly(phe) synthesis in the presence of 160 mg/l mocimycin, whereas a residual activity of 50% for the parent SrEF-Tu from S. ramocissimus CBS 190.69 was already observed at 1.6 mg/l mocimycin (Figure 4a).

In the second experiment, the synthesis of poly(phe) over a period of 40 minutes at a mocimycin concentration of 16 mg/l was studied. It was found that during this incubation, $^3$H-phenylalanine incorporation directed by both SrEF-Tu mutants A378V and A378T, proceeds with an efficiency of 80% if compared to the parallel incubation without mocimycin. In the control experiment, S-30 extracts containing parent SrEF-Tu in the presence of 16 mg/l mocimycin performed with a maximum efficiency of 20% of the mocimycin free reaction (Figure 4b).

Example 6

Visualization of elfamycin binding of SrEF-Tu mutants A378V, A378T, A378P, and A378F by non-denaturing PAGE

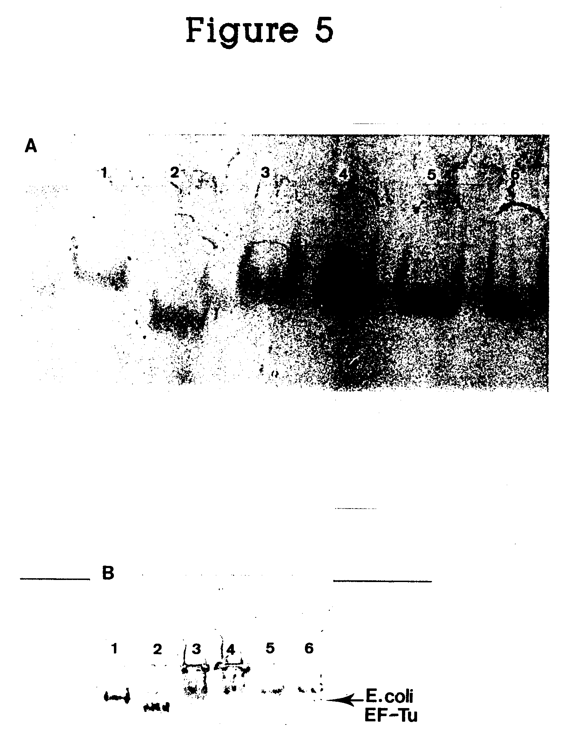

For direct visualization of the elfamycin binding capacity, the mutant proteins were expressed in E. coli JM101 essentially as described in Example 3. Subsequently, a GDP stabilized S-30, S-100, or purified SrEF-Tu sample was prepared, and incubated with 25 $\mu$M aurodox for 15 minutes at 37°C. These samples, and control samples without aurodox were then subjected to non-denaturing 10% polyacrylamide gels and electrophoresed. Detection of the SrEF-Tu species was performed by the Western blotting technique using SrEF-Tu antibodies (Example 1). Whereas wild-type S. ramocissimus EF-Tu appears to bind the elfamycin as indicated by the increased migration of the ternary complex into the gel (Figure 5), the migration of the mutant SrEF-Tu proteins A378V, A378T, A378P, and A378F was unaffected by preincubation with the elfamycin. This experiment thus established that elfamycin resistance is most likely the effect of a reduced binding of the elfamycin to the mutant SrEF-Tu proteins.

Example 7

Construction of the gene replacement vectors pMTST1V$\Delta$S and pMTST1T$\Delta$S

In order to obtain plasmids pMTST1V$\Delta$S and pMTST1T$\Delta$S capable of replacing the parent chromosomal Srtuf gene, several intermediate constructs were prepared.

**pUt18:**

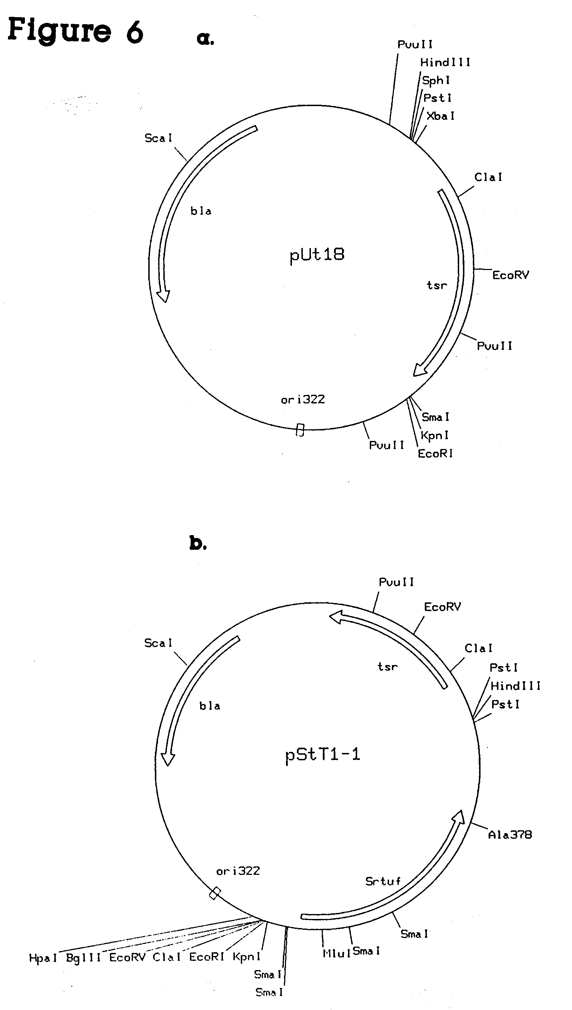

Plasmid pIJ702 (E. Katz et al., J. Gen. Microbiol. 129 (1983) 2703-2714) was digested with BcII, the 1.05 kb fragment containing the thiostrepton resistance gene was purified and subsequently ligated into BamHI digested pUC18. Transformation of E. coli JM101 yielded the desired plasmid pUt18 (Figure 6a).

**pStT1V-1 and pStT1T-1:**

pUt18 was digested with SmaI and HindIII and the 1.1 kb fragment containing the thiostrepton resistance gene was purified.

pUSrT1V-1 and pUSrT1T-1 were digested with EcoRI and HindIII and the 1.9 kb fragment containing the mutated Srtuf gene was purified.

Both purified fragments were combined with pSP70 (Promega) digested with PvuII and EcoRI, ligated and transformed to E. coli JM101. Plasmids containing all three of the above elements were identified and named pStT1V-1 and pStT1T-1, respectively (Figure 6b).

**pStT1V△S and pStT1T△S:**

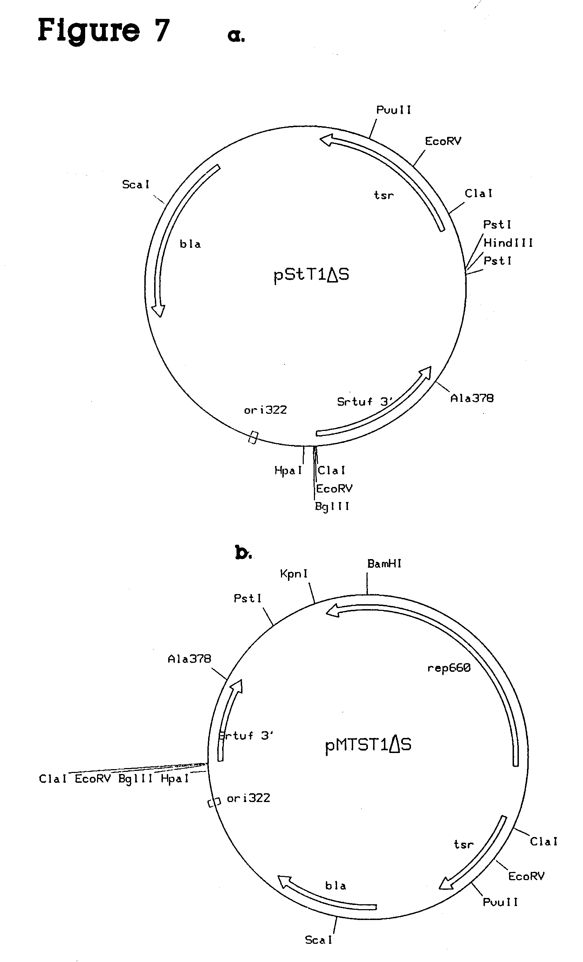

The upstream region and 5' coding region of the mutant Srtuf gene was deleted from plasmids pStT1V-1 and pStT1T-1 by digestion with EcoRI and SmaI, followed by treatment with DNA polymerase I (large fragment) to convert the sticky EcoRI ends to blunt ends, ligation, and transformation to E. coli JM101. The desired constructs were obtained and named pStT1V△S and pStT1T△S (Figure 7a).

**pMTST1V△S and pMTST1T△S:**

The larger fragments resulting from PstI/PvuII digestion of pStT1V△S and pMT660 (A. Birch and J. Cullum, J. Gen. Microbiol. 131 (1985) 1299-1303), respectively, were ligated and transformed to E. coli JM101. Thus plasmids pMTST1V△S was obtained. Similarly, starting from pStT1T△S, plasmid pMTST1T△S was constructed (Figure 7b).

Example 8

Replacement of the parent S. ramocissimus tuf1 gene by a mutated tuf1 gene encoding an elfamycin resistant EF-Tu protein

For replacement of the parent S. ramocissimus EF-Tu encoding gene by the mutant elfamycin resistant EF-Tu variant genes A378V and A378T, fresh spores of S. ramocissimus CBS 190.69 were prepared using sporulation medium of the following composition: $NaNO_3$ 0.3 g/l, $K_2HPO_4.3H_2O$ 0.2 g/l, $MgSO_4.7H_2O$ 0.2 g/l, $CaCl_2.2H_2O$ 0.005 g/l, $FeSO_4.7H_2O$ 0.01 g/l, $ZnSO_4.7H_2O$ 0.01 g/l, $CuSO_4.5H_2O$ 0.005 g/l, $MnSO_4.4H_2O$ 0.04 g/l, L-Methionine 0.1 g/l, L-Leucine 0.1 g/l, L-Tyrosine 0.5 g/l, glucose 10 g/l, and agar 20 g/l. Starting from a culture in S-medium (Example 1), 0.5 ml was spread on sporulation plates and incubated at 30°C for 5 days.

Spores were isolated essentially as described by D. Hopwood et al. (cited above), and used to inoculate YMG medium (yeast extract 4 g/l, malt extract 10 g/l, glucose 4 g/l) containing 0.5% glycine. Protoplasts were obtained by lysozyme treatment of this culture, transformed as described by D. Hopwood et al. (cited above) with plasmid pMTST1V△S and pMTST1T△S. Subsequently the transformed protoplasts were spread on regeneration medium, and incubated at 30°C. Regeneration medium was prepared by mixing equal volumes of sporulation medium and stabilizer medium. Stabilizer medium consisted of $NaNO_3$ 3 g/l, $K_2HPO_4.3H_2O$ 0.085 g/l, $K_2SO_4$ 0.25 g/l, $FeSO_4.7H_2O$ 0.01 g/l, trace element solution 0.1 ml, Tris 3.03 g/l, NaCl 2.92 g/l, sucrose 103 g/l, glucose 10 g/l, $MgCl_2.6H_2O$ 5 g/l, $CaCl_2.2H_2O$ 1.5 g/l, and agar 20 g/l, adjusted to pH 7.2 with 4N HCl. Trace element solution had the following composition: $Fe(NH_4)_2SO_4.6H_2O$ 0.25 g/l, $ZnSO_4.7H_2O$ 0.05 g/l, $MnCl_2.4H_2O$ 0.04 g/l, $CuSO_4.5H_2O$ 0.015 g/l, $CoCl_2.6H_2O$ 0.015 g/l, $H_3BO_3$ 0.005 g/l $NaMoO_4.2H_2O$ 0.0055 g/l, KI 0.01 g/l, adjusted to pH 3.0 with 4N HCl. After 24 hrs the regeneration plates were overlaid with 3 ml soft agar containing 20 $\mu$g/ml thiostrepton (D. Hopwood et al., cited above) and incubated at 30°C for 5 days.

Thiostrepton resistant colonies were streaked on sporulation medium containing 2 $\mu$g/ml thiostrepton, and individual colonies were cultured at 30°C in YMG medium containing 2 $\mu$g/ml thiostrepton. Subsequently plasmid DNA was isolated from each culture and analyzed by restriction enzyme mapping to confirm the identity and integrity of the transformed plasmids pMTST1VΔS and pMTST1TΔS.

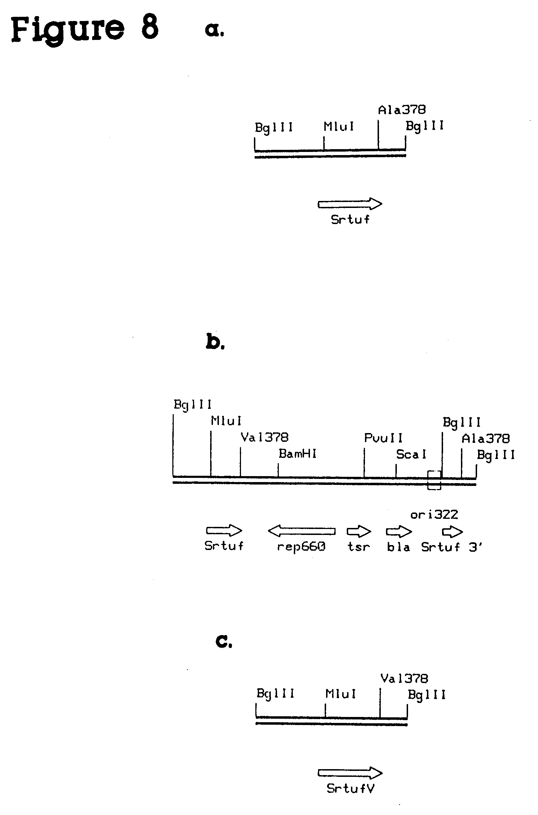

To obtain integration of plasmid pMTST1VΔS in the chromosome of S. ramocissimus CBS 190.69, preferably by homologous recombination of the plasmid located mutant SrtufR sequences with the parent Srtuf1 locus (Figure 8a), use was made of the temperature sensitive pMT660 replicon. Selected transformants were passed through several (at least 3) cycles of culturing in liquid medium (YMG) and sporulation at 37°C in the presence of 2 μg/ml thiostrepton, in order to remove freely replicating plasmid from the cells, but to select for chromosomal integration of the plasmid. Spores obtained by this procedure were diluted, plated, and incubated at 37°C; individual colonies were picked, grown at 37°C in YMG containing 2 μg/ml thiostrepton, and checked for the absence of plasmid DNA. Next, total DNA was isolated from plasmid free colonies, digested with BglII and analyzed by Southern blotting. Integration was observed through disappearance of the chromosomal 2.8 kb band and appearance of both a 1.2 and a 9.2 kb band (Figure 8b).

Strains having one plasmid copy integrated into the chromosomal Srtuf1 locus, were grown in YMG without thiostrepton and plated on non-selective sporulation medium. Spores were isolated, diluted, and plated on non-selective sporulation medium. Subsequent replica plating of single colonies to sporulation medium containing 2 μg/ml thiostrepton identified thiostrepton sensitive strains which had lost the plasmid sequences by intramolecular homologous recombination of the chromosome (the reverse process of plasmid integration). Selection of thiostrepton sensitive strains for elfamycin resistance both in liquid YMG medium containing 0.5 g/l mocimycin and on solid sporulation medium containing 0.1 g/l mocimycin yielded strain S. ramocissimus R1V having a restored chromosomal Srtuf locus which is identical to parent S. ramocissimus CBS 190.69, except for the A378V mutation (Figure 8c).

Similarly, plasmid pMTST1TΔS can be used to obtain S. ramocissimus strain R1T having the parent Srtuf locus, except for the mutation A378T.

Example 9

Elfamycin resistance properties of S.ramocissimus strain R1V

Spores, mycelium, and protoplasts of strain S. ramocissimus R1V were examined with respect to the minimal inhibitory concentration of mocimycin on their growth properties.

Spores of strain S. ramocissimus R1V and the control S. ramocissimus CBS 190.69 were inoculated at $5.10^7$ spores/ml in parallel shake flasks containing 25 ml YMG medium and mocimycin at concentrations ranging from 0 to 1 g/l. Incubation was for 5 days at 30°C. Table 1 illustrates the results of this experiment. For the control strain 0.15 g/l mocimycin inhibited germination (and/or growth) of the spores, whereas spores of S. ramocissimus strain R1V still germinated and grew at mocimycin concentrations up to 0.75 g/l.

Similar results were obtained on solid medium (HI-agar, Difco) containing 0 to 1 g/l mocimycin; spores from S. ramocissimus R1V and S. ramocissimus CBS 190.69 were diluted such that approximately 200 colony forming units were applied to each agar plate. Incubation of the plates was at 30°C for 5 days. For the control strain S. ramocissimus CBS 190.69 no colonies appeared above 0.1 g/l mocimycin; on the contrary, spores of S. ramocissimus strain R1V quantitatively were able to germinate and form colonies at least up to 0.75 g/l.

Essentially identical results were obtained when for each of the strains spores were substituted by mycelium, pregrown in YMG medium without mocimycin. Plating 1 ml of a 16 hrs culture it was found that strain S. ramocissimus R1V and the control S. ramocissimus CBS 190.69 had minimal inhibitory concentrations of 0.75 and 0.15 g/l, respectively.

Another test was carried out using protoplasts of strains S. ramocissimus CBS 190.69 and S. ramocissimus R1V, prepared as described in Example 7. As protoplasts lack the cell wall, which forms a protective barrier between the intracellular compartment and the external medium, they exhibit a considerably higher sensitivity towards elfamycin than do spores or mycelium. Thus protoplasts were plated on regeneration medium (Example 7) containing 0 to 0.1 g/l mocimycin. Under these conditions protoplasts of S. ramocissimu CBS 190.69 were able to regenerate only at mocimycin concentrations below 0.02 g/l; regeneration of S. ramocissimus R1V protoplasts in the presence of up to 0.1 g/l mocimycin occurred with the same efficiency as without mocimycin (Table 2).

## Table 1

Germination and growth of S. ramocissimus spores on HI-agar containing varying amounts of mocimycin.

| | Mocimycin concentration (g/l) | | | | | | |
|---|---|---|---|---|---|---|---|
| | 0 | 0.10 | 0.20 | 0.40 | 0.60 | 0.75 | 1.0 |
| S. ramocissimus CBS 169.90 | +++ | + | - | - | - | - | - |
| S.ramocissimus R1V | +++ | +++ | +++ | ++ | ++ | + | - |

+++ = good growth; ++ = slow growth; + = very slow growth; - = no growth

## Table 2

Regeneration efficiency of S. ramocissimus protoplasts on regeneration medium at different mocimycin concentrations.

| | Mocimycin concentration (g/l) | | | | | |
|---|---|---|---|---|---|---|
| | 0.00 | 0.01 | 0.02 | 0.04 | 0.07 | 0.10 |
| S. ramocissimus CBS 190.69 | 100% | 40% | 0% | 0% | 0% | 0% |
| S. ramocissimus R1V | 100% | 100% | 100% | 100% | 90% | 80% |

Example 10

Analysis of EF-TuR isolated from S. ramocissimus strain R1V

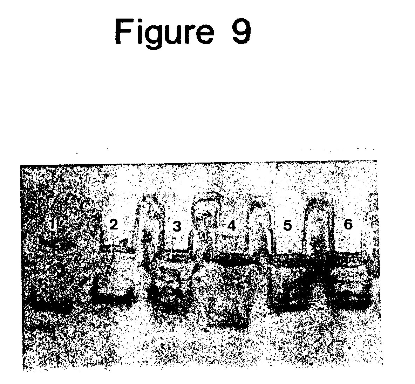

To establish that S. ramocissimus strain R1V actually expresses the mutated Srtuf1 gene as the main EF-Tu species, an S-100 extract was prepared from a culture of S. ramocissimus strain R1V essentially as described in Example 1. Subsequently this extract was subjected to the direct elfamycin binding assay outlined in Example 6. The results of this experiment, shown in Figure 9, prove that the major EF-Tu species of S. ramocissimus strain R1V contrary to that of strain CBS 190.69 is unable to bind the elfamycin aurodox under the conditions employed.

SEQUENCE LISTING

SEQ ID NO : 1

SEQUENCE TYPE : Nucleotide with corresponding protein

SEQUENCE LENGTH : 1194 base pairs

STRANDEDNESS : double-stranded

TOPOLOGY : Linear

MOLECULE TYPE : Genomic DNA

ORIGINAL SOURCE : Streptomyces ramocissimus

STRAIN : CBS 190.69

FEATURES : from 1 to 1191 bp: coding sequence

        from 1 to 396 aa : translation elongation factor Tu1 protein

PROPERTY :    Streptomyces ramocissimus tuf1 gene, encoding translation elongation factor Tu1

```
GTG GCG AAG GCG AAG TTC GAG CGG ACT AAG CCG CAC GTC AAC ATC GGC      48
Ala Lys Ala Lys Phe Glu Arg Thr Lys Pro His Val Asn Ile Gly
 1               5                   10                  15

ACC ATC GGT CAC ATC GAC CAC GGT AAG ACG ACC CTC ACG GCC GCC ATT      96
Thr Ile Gly His Ile Asp His Gly Lys Thr Thr Leu Thr Ala Ala Ile
              20                  25                  30

ACC AAG GTG CTG CAC GAC GCG TAC CCG GAC CTG AAC GAG GCC ACC CCG     144
Thr Lys Val Leu His Asp Ala Tyr Pro Asp Leu Asn Glu Ala Thr Pro
              35                  40                  45

TTC GAC AAC ATC GAC AAG GCT CCT GAG GAG CGT CAG CGC GGT ATC ACC     192
Phe Asp Asn Ile Asp Lys Ala Pro Glu Glu Arg Gln Arg Gly Ile Thr
              50                  55                  60

ATC TCC ATC GCG CAC GTC GAG TAC CAG ACC GAG GCG CGT CAC TAC GCC     240
Ile Ser Ile Ala His Val Glu Tyr Gln Thr Glu Ala Arg His Tyr Ala
              65                  70                  75

CAC GTC GAC TGC CCG GGT CAC GCG GAC TAC ATC AAG AAC ATG ATC ACG     288
His Val Asp Cys Pro Gly His Ala Asp Tyr Ile Lys Asn Met Ile Thr
 80                  85                  90                  95

GGT GCG GCG CAG ATG GAC GGC GCC ATC CTC GTG GTC GCC GCC ACC GAC     336
Gly Ala Ala Gln Met Asp Gly Ala Ile Leu Val Val Ala Ala Thr Asp
                   100                 105                 110

GGC CCG ATG CCG CAG ACC AAG GAG CAC GTG CTC CTG GCC CGC CAG GTC     384
Gly Pro Met Pro Gln Thr Lys Glu His Val Leu Leu Ala Arg Gln Val
                   115                 120                 125
```

13

```
GGC GTT CCG TAC ATC GTG GTC GCC CTG AAC AAG GCC GAC ATG GTG GAC        432
Gly Val Pro Tyr Ile Val Val Ala Leu Asn Lys Ala Asp Met Val Asp
        130             135             140

GAC GAG GAG ATC ATG GAG CTC GTT GAG CTC GAG GTC CGT GAG CTC CTC        480
Asp Glu Glu Ile Met Glu Leu Val Glu Leu Glu Val Arg Glu Leu Leu
        145             150             155

TCC GAG TAC GAG TTC CCG GGC GAC GAC CTG CCG GTC GTC CGC GTC TCC        528
Ser Glu Tyr Glu Phe Pro Gly Asp Asp Leu Pro Val Val Arg Val Ser
160             165             170             175

GCG CTG AAG GCG CTG GAG GGC GAC GCT CAG TGG ACG CAG TCC GTC CTC        576
Ala Leu Lys Ala Leu Glu Gly Asp Ala Gln Trp Thr Gln Ser Val Leu
        180             185             190

GAC CTG ATG AAG GCC GTC GAC GAG TCC ATC CCG GAG CCG GAG CGC GAC        624
Asp Leu Met Lys Ala Val Asp Glu Ser Ile Pro Glu Pro Glu Arg Asp
        195             200             205

GTC GAC AAG CCG TTC CTC ATG CCG ATC GAG GAC GTC TTC ACG ATC ACC        672
Val Asp Lys Pro Phe Leu Met Pro Ile Glu Asp Val Phe Thr Ile Thr
        210             215             220

GGT CGC GGC ACG GTC GTC ACC GGC CGT ATC GAG CGT GGT GTC CTG AAG        720
Gly Arg Gly Thr Val Val Thr Gly Arg Ile Glu Arg Gly Val Leu Lys
        225             230             235

GTC AAC GAG ACC GTC GAC ATC ATC GGC ATC AAG ACC GAG AAG ACC ACC        768
Val Asn Glu Thr Val Asp Ile Ile Gly Ile Lys Thr Glu Lys Thr Thr
240             245             250             255

ACC ACG GTC ACC GGC ATC GAG ATG TTC CGC AAG CTG CTC GAC GAG GGC        816
Thr Thr Val Thr Gly Ile Glu Met Phe Arg Lys Leu Leu Asp Glu Gly
        260             265             270

CAG GCC GGT GAG AAC GTC GGT CTG CTG CTC CGC GGC ATC AAG CGC GAG        864
Gln Ala Gly Glu Asn Val Gly Leu Leu Leu Arg Gly Ile Lys Arg Glu
        275             280             285

GAC GTC GAG CGC GGC CAG GTC ATC ATC AAG CCG GGC TCG GTC ACC CCG        912
Asp Val Glu Arg Gly Gln Val Ile Ile Lys Pro Gly Ser Val Thr Pro
        290             295             300

CAC ACC GAG TTC GAG GCG CAG GCC TAC ATC CTC TCC AAG GAC GAG GGT        960
His Thr Glu Phe Glu Ala Gln Ala Tyr Ile Leu Ser Lys Asp Glu Gly
        305             310             315

GGC CGC CAC ACG CCG TTC TTC AAC AAC TAC CGC CCG CAG TTC TAC TTC       1008
Gly Arg His Thr Pro Phe Phe Asn Asn Tyr Arg Pro Gln Phe Tyr Phe
320             325             330             335

CGT ACC ACG GAC GTG ACC GGC GTT GTG CAC CTC CCC GAG GGC ACC GAG       1056
Arg Thr Thr Asp Val Thr Gly Val Val His Leu Pro Glu Gly Thr Glu
        340             345             350
```

```
ATG GTC ATG CCG GGC GAC AAC ACC GAG ATG CGC GTC GAG CTG ATC CAG      1104
Met Val Met Pro Gly Asp Asn Thr Glu Met Arg Val Glu Leu Ile Gln
            355                 360                 365

CCC GTC GCC ATG GAG GAG GGC CTG AAG TTC GCC ATC CGT GAG GGT GGC      1152
Pro Val Ala Met Glu Glu Gly Leu Lys Phe Ala lle Arg Glu Gly Gly
            370                 375                 380

CGG ACC GTC GGC GCC GGC CAG GTC ACC AAG ATC GTC AAG TAA              1194
Arg Thr Val Gly Ala Gly Gln Val Thr Lys Ile Val Lys
    385                 390                 395
```

SEQ ID NO : 2

SEQUENCE TYPE : Nucleotide with corresponding protein

SEQUENCE LENGTH : 1194 base pairs

STRANDEDNESS : double-stranded

TOPOLOGY : Linear

MOLECULE TYPE : Genomic DNA

ORIGINAL SOURCE : Streptomyces ramocissimus

STRAIN : CBS 190.69

FEATURES : from 1 to 1191 bp: coding sequence

           from 1 to 396 aa : translation elongation factor Tu2 protein

PROPERTY :    Streptomyces ramocissimus   tuf2  gene,  encoding  translation

           elongation factor Tu2

```
GTG GCG AAG GCG AAG TTC CAG CGG ACC AAA CCG CAC GTC AAC ATC GGC        48
    Ala Lys Ala Lys Phe Gln Arg Thr Lys Pro His Val Asn Ile Gly
    1               5               10              15

ACC ATC GGC CAC ATC GAC CAC GGC AAG ACG ACA CTC ACC GCG GCG ATC        96
Thr Ile Gly His Ile Asp His Gly Lys Thr Thr Leu Thr Ala Ala Ile
            20              25              30

ACG AAG GTG CTG CAC GAC CGG TTC CCC GAC CTC AAC CCG TTC ACC CCG       144
Thr Lys Val Leu His Asp Arg Phe Pro Asp Leu Asn Pro Phe Thr Pro
            35              40              45

TTC GAC CAG ATC GAC AAG GCG CCC GAG GAA CGG CAG CGC GGC ATC ACC       192
Phe Asp Gln Ile Asp Lys Ala Pro Glu Glu Arg Gln Arg Gly Ile Thr
            50              55              60

ATC TCG ATC GCC CAC GTC GAG TAC CAG ACC GAG GCG CGG CAC TAC GCG       240
Ile Ser Ile Ala His Val Glu Tyr Gln Thr Glu Ala Arg His Tyr Ala
    65              70              75

CAC GTC GAC TGC CCC GGA CAC GCC GAC TAC ATC AAG AAC ATG ATC ACC       288
His Val Asp Cys Pro Gly His Ala Asp Tyr Ile Lys Asn Met Ile Thr
80              85              90              95

GGC GCG GCC CAG ATG GAC GGC GCG ATC CTG GTC GTC GCG GCC ACG GAC       336
Gly Ala Ala Gln Met Asp Gly Ala Ile Leu Val Val Ala Ala Thr Asp
                100             105             110

GGG CCG ATG CCC CAG ACC AAG GAA CAT GTG CTG CTG GCA CGG CAG GTG       384
Gly Pro Met Pro Gln Thr Lys Glu His Val Leu Leu Ala Arg Gln Val
                115             120             125
```

```
GGC GTG CCC TAC ATC GTC GTC GCG CTG AAC AAG ACC GAC ATG GTC GAC      432
Gly Val Pro Tyr Ile Val Val Ala Leu Asn Lys Thr Asp Met Val Asp
        130                 135                 140

GAC GAG GAG ATC CTC GAA CTC GTG GAG TTG GAG GTG CGC GAG CTG CTC      480
Asp Glu Glu Ile Leu Glu Leu Val Glu Leu Glu Val Arg Glu Leu Leu
        145                 150                 155

ACC GAG TAC GAG TTC CCC GGC GAC GAC GTC CCG GTC GTC AAG GTG TCG      528
Thr Glu Tyr Glu Phe Pro Gly Asp Asp Val Pro Val Val Lys Val Ser
160                 165                 170                 175

GCG CTC AGG GCC CTG GAG GGC GAC CCC CGG TGG ACC CGG TCG GTA CTC      576
Ala Leu Arg Ala Leu Glu Gly Asp Pro Arg Trp Thr Arg Ser Val Leu
                180                 185                 190

GAA CTC CTC GAC GCC GTC GAC GAG TTC GTG CCC GAG CCG GTG CGG GAC      624
Glu Leu Leu Asp Ala Val Asp Glu Phe Val Pro Glu Pro Val Arg Asp
            195                 200                 205

GTC GAC CGG CCG TTC CTG ATG CCG ATC GAG GAC GTC TTC ACC ATC ACC      672
Val Asp Arg Pro Phe Leu Met Pro Ile Glu Asp Val Phe Thr Ile Thr
        210                 215                 220

GGA CGC GGC ACG GTC GTC ACC GGC CGG ATA GAG CGC GGC ACG CTG AAC      720
Gly Arg Gly Thr Val Val Thr Gly Arg Ile Glu Arg Gly Thr Leu Asn
        225                 230                 235

GTG AAC ACC GAG GTG GAG ATC ATC GGC ATC CAC GAA CAG AGG ACC CGG      768
Val Asn Thr Glu Val Glu Ile Ile Gly Ile His Glu Gln Arg Thr Arg
240                 245                 250                 255

ACC ACG GTC ACC GGC ATC GAG ATG TTC CGC AAG CTC CTC GAC GAG GGC      816
Thr Thr Val Thr Gly Ile Glu Met Phe Arg Lys Leu Leu Asp Glu Gly
                260                 265                 270

CGG GCC GGC GAG AAC GTC GGA CTG CTG CTG CGC GGA GTG AAG CGG GAG      864
Arg Ala Gly Glu Asn Val Gly Leu Leu Leu Arg Gly Val Lys Arg Glu
                275                 280                 285

CAG GTC GAG CGC GGT CAG GTC GTC ATC AGG CCC GGA TCG GTC ACC CCG      912
Gln Val Glu Arg Gly Gln Val Val Ile Arg Pro Gly Ser Val Thr Pro
        290                 295                 300

CAC ACG CAG TTC GAG GCG CAG GCG TAC ATC CTG TCC AAG GAC GAG GGC      960
His Thr Gln Phe Glu Ala Gln Ala Tyr Ile Leu Ser Lys Asp Glu Gly
        305                 310                 315

GGC CGG CAC ACG CCG TTC TTC GAG AAC TAC CGT CCG CAG TTC TAC TTC     1008
Gly Arg His Thr Pro Phe Phe Glu Asn Tyr Arg Pro Gln Phe Tyr Phe
320                 325                 330                 335

CGC ACC ACC GAC GTC ACG GGC GTG GTG ACG CTG CCG AAG GGC ACC GAG     1056
Arg Thr Thr Asp Val Thr Gly Val Val Thr Leu Pro Lys Gly Thr Glu
                340                 345                 350
```

17

```
ATG GTG ATG CCG GGC GAC AAC ACC GCC ATG CAC GTC CAG CTG ATC CAG          1104
Met Val Met Pro Gly Asp Asn Thr Ala Met His Val Gln Leu Ile Gln
            355                 360                 365


CCG ATC GCC ATG GAG GAG GGG CTG AAG TTC GCC ATC CGC GAG GGC GGG          1152
Pro Ile Ala Met Glu Glu Gly Leu Lys Phe Ala Ile Arg Glu Gly Gly
        370                 375                 380

CGC ACG GTC GGC GCC GGC CAG GTC ACG CGG ATC GTG AAG TAG              1194
Arg Thr Val Gly Ala Gly Gln Val Thr Arg Ile Val Lys
    385                 390                 395
```

SEQ ID NO : 3

SEQUENCE TYPE : Nucleotide with corresponding protein

SEQUENCE LENGTH : 1170 base pairs

STRANDEDNESS : double-stranded

TOPOLOGY : Linear

MOLECULE TYPE : Genomic DNA

ORIGINAL SOURCE : Streptomyces ramocissimus

STRAIN : CBS 190.69

FEATURES : from 1 to 1167 bp: coding sequence

from 1 to 388 aa : translation elongation factor Tu3 protein

PROPERTY :     Streptomyces     ramocissimus     tuf3     gene,     encoding     translation
elongation factor Tu3

```
ATG TCC AAG ACG GCA TAC GTG CGC ACC AAA CCG CAT CTG AAC ATC GGC        48
    Ser Lys Thr Ala Tyr Val Arg Thr Lys Pro His Leu Asn Ile Gly
    1               5               10              15

ACG ATG GGT CAT GTC GAC CAC GGC AAG ACC ACG TTG ACC GCC GCC ATC        96
Thr Met Gly His Val Asp His Gly Lys Thr Thr Leu Thr Ala Ala Ile
                20              25              30

ACC AAG GTC CTC GCC GAG CGT GGC TCC GGG ACG TTC GTC CCG TTC GAC       144
Thr Lys Val Leu Ala Glu Arg Gly Ser Gly Thr Phe Val Pro Phe Asp
            35              40              45

CGC ATC GAC CGG GCC CCG GAG GAG GCC GCG CGC GGC ATC ACC ATC AAC       192
Arg Ile Asp Arg Ala Pro Glu Glu Ala Ala Arg Gly Ile Thr Ile Asn
        50              55              60

ATC GCG CAC GTC GAG TAC GAG ACC GAC ACC CGG CAC TAC GCG CAC GTC       240
Ile Ala His Val Glu Tyr Glu Thr Asp Thr Arg His Tyr Ala His Val
        65              70              75

GAC ATG CCG GGC CAC GCC GAC TAC GTC AAG AAC ATG GTC ACC GGC GCC       288
Asp Met Pro Gly His Ala Asp Tyr Val Lys Asn Met Val Thr Gly Ala
80              85              90              95

GCG CAG CTC GAC GGG GCG ATC CTC GTC GTC TCC GCG CTC GAC GGG ATC       336
Ala Gln Leu Asp Gly Ala Ile Leu Val Val Ser Ala Leu Asp Gly Ile
            100             105             110

ATG CCG CAG ACC GCC GAA CAC GTC CTG CTC GCC CGG CAG GTG GGC GTC       384
Met Pro Gln Thr Ala Glu His Val Leu Leu Ala Arg Gln Val Gly Val
            115             120             125
```

```
GAC CAC ATC GTC GTC GCC CTC AAC AAG GCC GAC GCG GGC GAC GAG GAG        432
Asp His Ile Val Val Ala Leu Asn Lys Ala Asp Ala Gly Asp Glu Glu
        130               135              140

CTC ACC GAC CTC GTC GAG CTG GAG GTC CGC GAT CTG CTC TCC GAG CAC        480
Leu Thr Asp Leu Val Glu Leu Glu Val Arg Asp Leu Leu Ser Glu His
        145               150              155

GGC TAC GGC GGC GAC GGT GCC CCC GTC GTA CGG GTC TCG GGG CTG AAG        528
Gly Tyr Gly Gly Asp Gly Ala Pro Val Val Arg Val Ser Gly Leu Lys
160               165               170               175

GCG CTG GAG GGC GAC CCC AAG TGG ACG GCG TCC ATC GAG GCG CTG CTC        576
Ala Leu Glu Gly Asp Pro Lys Trp Thr Ala Ser Ile Glu Ala Leu Leu
                180               185               190

GAC GCG GTG GAC ACC TAC GTG CCG ATG CCG GAG CGG TAT GTG GAC GCG        624
Asp Ala Val Asp Thr Tyr Val Pro Met Pro Glu Arg Tyr Val Asp Ala
                195               200               205

CCG TTC CTG CTG CCC GTG GAG AAC GTG CTC ACC ATC ACC GGT CGG GGG        672
Pro Phe Leu Leu Pro Val Glu Asn Val Leu Thr Ile Thr Gly Arg Gly
                210               215               220

ACC GTG GTC ACC GGA GCC GTC GAG CGG GGC ACC GTG CGC GTG GGC AAC        720
Thr Val Val Thr Gly Ala Val Glu Arg Gly Thr Val Arg Val Gly Asn
        225               230               235

CGG GTC GAA GTG CTC GGC GCC GGG CTG GAG ACC GTG GTC ACC GGC CTG        768
Arg Val Glu Val Leu Gly Ala Gly Leu Glu Thr Val Val Thr Gly Leu
240               245               250               255

GAG ACG TTC GGC AAG CCC ATG GAC GAG GCG CAG GCC GGG GAC AAC GTG        816
Glu Thr Phe Gly Lys Pro Met Asp Glu Ala Gln Ala Gly Asp Asn Val
                260               265               270

GCG CTG TTG CTG CGT GGG GTT CCG CGG GAC GCC GTA CGG CGT GGG CAT        864
Ala Leu Leu Leu Arg Gly Val Pro Arg Asp Ala Val Arg Arg Gly His
                275               280               285

GTG GTC GCG GCG CCG GGG AGC GTG GTG CCC CGG AGT CGA TTC TCC GCG        912
Val Val Ala Ala Pro Gly Ser Val Val Pro Arg Ser Arg Phe Ser Ala
                290               295               300

CAG GTG TAT GTG CTC TCG GCC CGC GAG GGC GGT CGT ACG ACT CCT GTC        960
Gln Val Tyr Val Leu Ser Ala Arg Glu Gly Gly Arg Thr Thr Pro Val
        305               310               315

ACC AGC GGG TAT CGG CCG CAG TTC TAC ATC CGT ACG GCG GAT GTG GTG       1008
Thr Ser Gly Tyr Arg Pro Gln Phe Tyr Ile Arg Thr Ala Asp Val Val
320               325               330               335

GGG GAC GTC GAC CTG GGG GAG GTG GGG GTC GCT CGG CCT GGG GAG ACG       1056
Gly Asp Val Asp Leu Gly Glu Val Gly Val Ala Arg Pro Gly Glu Thr
                340               345               350
```

```
GTT TCG ATG ATC GTC GAG TTG GGC CGG GAG GTT CCG CTG GAG CCC GGG     1104
Val Ser Met Ile Val Glu Leu GIy Arg Glu Val Pro Leu Glu Pro Gly
        355                 360                 365

TTG GGG TTC GCC ATT CGT GAG GGC GGC AGG ACC GTG GGG GCG GGG ACC     1152
Leu Gly Phe Ala Ile Arg Glu Gly Gly Arg Thr Val Gly Ala Gly Thr
        370                 375                 380

GTT ACG GCC CTT GTG TGA                                             1170
Val Thr Ala Leu Val
    385
```

SEQ ID NO : 4

SEQUENCE TYPE : Nucleotide

SEQUENCE LENGTH : 33 nucleotides

STRANDEDNESS : single-stranded

TOPOLOGY : Linear

MOLECULE TYPE : Synthetic DNA

PROPERTY :    Oligonucleotide changing the codon for alanine 378 of the S.ramocissimus Srtuf1 gene to valine.

GCCACCCTCA CGGATGACGA ACTTCAGGCC CTC        33

SEQ 1D NO : 5

SEQUENCE TYPE : Nucleotide

SEQUENCE LENGTH : 33 nucleotides

STRANDEDNESS : single-stranded

TOPOLOGY : Linear

MOLECULE TYPE : Synthetic DNA

PROPERTY :    Oligonucleotide changing the codon for alanine 378 of the S.ramocissimus Srtuf1 gene to threonine.

GCCACCCTCA CGGATGGTGA ACTTCAGGCC CTC        33

SEQ ID NO : 6

SEQUENCE TYPE : Nucleotide

SEQUENCE LENGTH : 33 nucleotides

STRANDEDNESS : single-stranded

TOPOLOGY : Linear

MOLECULE TYPE : Synthetic DNA

PROPERTY :       Oligonucleotide  changing  the  codon  for  alanine  378  of  the
                 S.ramocissimus Srtuf1 gene to proline.


GCCACCCTCA CGGATCGGGA ACTTCAGGCC CTC        33


SEQ ID NO : 7

SEQUENCE TYPE : Nucleotide

SEQUENCE LENGTH : 33 nucleotides

STRANDEDNESS : single-stranded

TOPOLOGY : Linear

MOLECULE TYPE : Synthetic DNA

PROPERTY :       Oligonucleotide  changing  the  codon  for  alanine  378  of  the
                 S.ramocissimus Srtuf1 gene to phenylalanine.


GCCACCCTCA CGGATGAAGAA CTTCAGGCC CTC        33


## Claims

1.  Protein EF-TuR, characterized in that it is obtainable from an elfamycin producing actinomycete and made elfamycin resistant.

2.  Protein EF-TuR according to Claim 1, characterized in that the elfamycin is mocimycin (kirromycin).

3.  Protein EF-TuR according to Claim 1 or 2, characterized in that, when tested in a cell-free system for poly (U)-directed polyphenylalanine synthesis, it has a residual activity of 50% in a medium containing at least 2 mg per liter mocimycin, preferably at least 160 mg per liter mocymicin.

4.  Protein EF-TuR according to any of Claims 1-3, characterized in that the actinomycete is a streptomycete, preferably Streptomyces ramocissimus, more preferably Streptomyces ramocissimus CBS 190.69.

5.  Protein EF-TuR according to any of Claims 1-4, consisting of an amino acid sequence corresponding by at least 80% to that depicted in Figure 1 of the accompanying drawings.

6.  Protein EF-TuR according to Claim 5, characterized in that the alanine at position 378 or at the position corresponding thereto in an homologous protein, is replaced by either valine, threonine, proline, or phenylalanine.

7. A DNA sequence tufR encoding protein EF-TuR according to any one of Claims 1-6.

8. A DNA sequence tufR according to Claim 7, as depicted in Figure 1 of the drawings, characterized in that the codon encoding the alanine at position 378, or at the position corresponding thereto in an homologous gene, is replaced by one encoding valine, threonine, proline, or phenylalanine.

9. A vector containing a DNA sequence according to Claim 7 or 8, preferably the vector is plasmid.

10. A plasmid vector characterized in that it is pMaSrT1V, pMaSrT1T, pMaSrT1P, pMaSrT1F, pUSrT1V-1, pUSrT1T-1, pUSrT1P-1, pUSrT1F-1, pStT1V-1, pStT1T-1, pStT1V∆S, pStT1T∆S, pMTST1V∆S, or pMTST1T∆S, as depicted in Figures 2, 3, 6, and 7 of the accompanying drawings.

11. An elfamycin producing actinomycete, comprising a DNA sequence tufR according to Claim 7 or 8.

12. An elfamycin producing actinomycete according to Claim 11, characterized in that the elfamycin is mocimycin.

13. An elfamycin producing actinomycete according to Claim 11 or 12, wherein said DNA sequence tufR is integrated in the chromosome, replacing the DNA sequence tuf.

14. An elfamycin producing actinomycete according to any of Claims 11-13, characterized in that it is a streptomycete.

15. A mocimycin producing streptomycete characterized in that its spores germinate and grow in YMG medium containing yeast extract 4 g/1, malt extract 10 g/l and glucose 4 g/l, in the presence of at least 0.2 g/l preferably 0.2-1.0 g/l mocymicin.

16. A streptomycete according to Claim 15, characterized in that it belongs to the species Streptomyces ramocissimus.

17. Streptomyces ramocissimus strain R1V derived from Streptomyces ramocissimus CBS 190.69, expressing the gene encoding the mutant protein SrEF-Tu A378V.

18. Process for obtaining an elfamycin producing actinomycete expressing an elfamycin resistant EF-TuR according to any of Claims 1-6, comprising the following steps:
1. cloning of the gene tuf from an elfamycin producing actinomycete,
2. applying site-directed mutagenesis on said gene tuf, thereby altering the gene tuf encoding an elfamycin sensitive EF-Tu into the gene tufR encoding an elfamycin resistant EF-TuR,
3. constructing a vector containing the gene tufR or a part thereof,
4. transforming an elfamycin producing actinomycete by introducing said vector into it,
5. selecting said transformant for integration of said vector into the chromosomal tuf locus, by its elfamycin resistant phenotype.

19. A process for the preparation of an elfamycin comprising the fermentation of an actinomycete capable of producing an elfamycin and which actinomycete is resistant to an elfamycin concentration of at least 0.2 g/l, preferably 0.2-1.0 g/l.

20. A process according to Claim 19 characterized in that the elfamycin is mocymicin.

21. A process according to Claim 19 or 20 characterized in that the actinomycete is a streptomycete, preferably Streptomyces ramocissimus

Figure 1

```
              .              .            30            .              .            60
GTGGCGAAGGCGAAGTTCGAGCGGACTAAGCCGCACGTCAACATCGGCACCATCGGTCAC
     A   K   A   K   F   E   R   T   K   P   H   V   N   I   G   T   I   G   H
     1

              .              .            90            .              .           120
ATCGACCACGGTAAGACGACCCTCACGGCCGCCATTACCAAGGTGCTGCACGACGCGTAC
  I   D   H   G   K   T   T   L   T   A   A   I   T   K   V   L   H   D   A   Y
 20

              .              .           150            .              .           180
CCGGACCTGAACGAGGCCACCCCGTTCGACAACATCGACAAGGCTCCTGAGGAGCGTCAG
  P   D   L   N   E   A   T   P   F   D   N   I   D   K   A   P   E   E   R   Q
 40

              .              .           210            .              .           240
CGCGGTATCACCATCTCCATCGCGCACGTCGAGTACCAGACCGAGGCGCGTCACTACGCC
  R   G   I   T   I   S   I   A   H   V   E   Y   Q   T   E   A   R   H   Y   A
 60

              .              .           270            .              .           300
CACGTCGACTGCCCGGGGTCACGCGGACTACATCAAGAACATGATCACGGGTGCGGCGCAG
  H   V   D   C   P   G   H   A   D   Y   I   K   N   M   I   T   G   A   A   Q
 80

              .              .           330            .              .           360
ATGGACGGCGCCATCCTCGTGGTCGCCGCCACCGACGGCCCCGATGCCGCAGACCAAGGAG
  M   D   G   A   I   L   V   V   A   A   T   D   G   P   M   P   Q   T   K   E
100

              .              .           390            .              .           420
CACGTGCTCCTGGCCCGCCAGGTCGGCGTTCCGTACATCGTGGTCGCCCTGAACAAGGCC
  H   V   L   L   A   R   Q   V   G   V   P   Y   I   V   V   A   L   N   K   A
120

              .              .           450            .              .           480
GACATGGTGGACGACGAGGAGATCATGGAGCTCGTTGAGCTCGAGGTCCGTGAGCTCCTC
  D   M   V   D   D   E   E   I   M   E   L   V   E   L   E   V   R   E   L   L
140

              .              .           510            .              .           540
TCCGAGTACGAGTTCCCGGGCGACGACCTGCCGGTCGTCCGCGTCTCCGCGCTGAAGGCG
  S   E   Y   E   F   P   G   D   D   L   P   V   V   R   V   S   A   L   K   A
160

              .              .           570            .              .           600
CTGGAGGGCGACGCTCAGTGGACGCAGTCCGTCCTCGACCTGATGAAGGCCGTCGACGAG
  L   E   G   D   A   Q   W   T   Q   S   V   L   D   L   M   K   A   V   D   E
180

              .              .           630            .              .           660
TCCATCCCGGAGCCGGAGCGCGACGTCGACAAGCCGTTCCTCATGCCGATCGAGGACGTC
  S   I   P   E   P   E   R   D   V   D   K   P   F   L   M   P   I   E   D   V
200
```

24

```
            .           .           690         .           .           720
TTCACGATCACCGGTCGCGGCACGGTCGTCACCGGCCGTATCGAGCGTGGTGTCCTGAAG
  F   T   I   T   G   R   G   T   V   V   T   G   R   I   E   R   G   V   L   K
220

            .           .           750         .           .           780
GTCAACGAGACCGTCGACATCATCGGCATCAAGACCGAGAAGACCACCACCACGGTCACC
  V   N   E   T   V   D   I   I   G   I   K   T   E   K   T   T   T   T   V   T
240

            .           .           810         .           .           840
GGCATCGAGATGTTCCGCAAGCTGCTCGACGAGGGCCAGGCCGGTGAGAACGTCGGTCTG
  G   I   E   M   F   R   K   L   L   D   E   G   Q   A   G   E   N   V   G   L
260

            .           .           870         .           .           900
CTGCTCCGCGGCATCAAGCGCGAGGACGTCGAGCGCGGCCAGGTCATCATCAAGCCGGGC
  L   L   R   G   I   K   R   E   D   V   E   R   G   Q   V   I   I   K   P   G
280

            .           .           930         .           .           960
TCGGTCACCCCGCACACCGAGTTCGAGGCGCAGGCCTACATCCTCTCCAAGGACGAGGGT
  S   V   T   P   H   T   E   F   E   A   Q   A   Y   I   L   S   K   D   E   G
300

            .           .           990         .           .           1020
GGCCGCCACACGCCGTTCTTCAACAACTACCGCCCGCAGTTCTACTTCCGTACCACGGAC
  G   R   H   T   P   F   F   N   N   Y   R   P   Q   F   Y   F   R   T   T   D
320

            .           .           1050        .           .           1080
GTGACCGGCGTTGTGCACCTCCCCGAGGGCACCGAGATGGTCATGCCGGGCGACAACACC
  V   T   G   V   V   H   L   P   E   G   T   E   M   V   M   P   G   D   N   T
340

            .           .           1110        .           .           1140
GAGATGCGCGTCGAGCTGATCCAGCCCGTCGCCATGGAGGAGGGCCTGAAGTTCGCCATC
  E   M   R   V   E   L   I   Q   P   V   A   M   E   E   G   L   K   F   A   I
360                                                                     378

            .           .           1170        .           .
CGTGAGGGTGGCCGGACCGTCGGCGCCGGCCAGGTCACCAAGATCGTCAAGTAA
  R   E   G   G   R   T   V   G   A   G   Q   V   T   K   I   V   K   *
380
```

# Figure 2    a.

pUSrT1

b.

pUSrT1-1

# Figure 3   a.

# Figure 4 a.

POLY(U) DIRECTED POLY(PHE) SYNTHESIS

a=0 b=0.16 c=1.6 d=16 e=16α f=1600 mg/L

SrEF-Tu

SrEF-Tu A378V

SrEF-Tu A378T

mg/l mocimycin

# Figure 4    b.

POLY(U) DIRECTED POLY(PHE) SYNTHESIS

**a.**                      SrEF-Tu

**b.**                SrEF-Tu A378V

**c.**                SrEF-Tu A378T

TIME (MIN)

■ - MOCIMYCIN    ▨ + MOCIMYCIN 16 mg/l

29

# Figure 5

A

B

← E.coli
EF-Tu

# Figure 6   a.

b.

# Figure 7   a.

# Figure 8 a.

# b.

# c.

# Figure 9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | HEREDITY vol. 61, no. 2, 1988, EDINBURGH pages 291 - 292; WOUDT B. ET AL: 'Analysis of TUF-genes from Streptomyces ramocissimus ' * abstract * | 1-21 | C12N15/31 C12P21/02 C12N1/21 C12R1/465 C12P19/02 |
| Y | BIOCHIMIE vol. 69, no. 10, October 1987, PARIS VIJGENBOOM E. ET AL: 'Transfer of plasmid-bone TUF mutations to the chromosome as a genetic tool for studying the functionning of EF-TuA and EF-TuB in the E.coli cell " ' * the whole document * | 1-21 | |
| D,Y | EMBO JOURNAL. vol. 3, no. 1, 1984, EYNSHAM, OXFORD GB pages 113 - 120; DUISTERWINJEL F.J. ET AL: 'Specific alterations of the Ef-Tu polypeptide chain considered in the light of its three-dimensionnal structure ' * the whole document * | 1-21 | |
| D,X | FEMS MICROBIOLOGY LETTERS vol. 25, 1984, AMSTERDAM pages 121 - 124; GLÖCKNER C. ET AL: 'Mechanism of natural resistance to kirromycin type antibiotics in Actinomycetes ' * the whole document , especially page 123-124 * | 15-16, 19-21 | TECHNICAL FIELDS SEARCHED (Int. Cl.5) C07K C12N C12P |
| D,A | | 1-14, 17-18 | |
| X | US-A-3 927 211 (GIST-BROCADES N.V.) * column 8, line 21 - line 62; claim 3 * | 15-16, 19-21 | |

The present search report has been drawn up for all claims

| Place of search | Date of completion of the search | Examiner |
|---|---|---|
| THE HAGUE | 09 OCTOBER 1991 | LE CORNEC N.D.R. |